# EUROPEAN PATENT APPLICATION

(11) **EP 4 751 638 A1**
(43) Date of publication of application: **03.06.2026**
(21) Application number: 25218790.1
(22) Date of filing: 26.11.2025
(51) Int. Cl.: A61B 5/00, A61B 5/026

(54) **SYSTEMS AND METHODS FOR DETECTING AN AREA OF INTEREST IN AN OBJECT**

(30) Priority: 27.11.2024 US 202418961576
(71) Applicant: Teledyne Dalsa B.V., 5656 AE Eindhoven (NL)
(72) Inventor: SNYDER, Jonathan E, Park City, 84098 (US); NIKIEL, Anna Maria, 5614 CD Eindhoven (NL); VAN LIER, Monique Geertruida Josepha Theodora Bernadina, 3981GH Bunnik (NL); DE GROOT, Jeroen Emanuel, 1031 AA Amsterdam (NL); DEN HEETEN, Gerard Johan, 1077 JZ Amsterdam (NL)
(74) Representative: HGF

(57) **Abstract**

Systems and methods for detecting an area of interest in an object are provided. The method comprises acquiring first thermographic data of the object in a first state of compression, transitioning the object from the first state of compression to a second state of compression, acquiring second thermographic data of the object in the second state of compression, and determining an area of interest based on an analysis of the first thermographic data and the second thermographic data. The second state of compression is different than the first state of compression. Transitioning the object from the first state of compression to the second state of compression is accomplished by increasing or decreasing a compressive force applied to the object. A thermal imaging system and a method for detecting a cancerous lesion in breast tissue are also provided.

## Description

### FIELD

This disclosure relates generally to systems and methods for detecting an area of interest in an object.

### BACKGROUND

Diagnosing breast cancer involves various examination techniques directed to supporting early and accurate cancer detection, including, for example, x-ray imaging. There are challenges with identifying breast cancer early and efficiently.

### SUMMARY

One general aspect according to the present disclosure is directed to a method for detecting an area of interest in an object. The method comprises acquiring first thermographic data of the object at a first state of compression. The object is transitioned from the first state of compression to a second state of compression that is different than the first state of compression. Transitioning the object from the first state of compression to the second state of compression is accomplished by increasing or decreasing a compressive force applied to the object. Second thermographic data of the object is acquired in the second state of compression. An area of interest is determined based on an analysis of the first thermographic data and the second thermographic data.

Another general aspect of the present disclosure is directed to a thermal imaging system for detecting an area of interest in an object. The thermal imaging system comprises a thermal camera and a control circuit in signal communication with the thermal camera. The thermal camera is capable to acquire first thermographic data of the object at a first state of compression and acquire second thermographic data of the object at a second state of compression. The control circuit is capable to determine the area of interest based on an analysis of the first thermographic data and the second thermographic data.

Another general aspect of the present disclosure is directed to a method for detecting a cancerous lesion in breast tissue. The method comprises disposing the breast tissue intermediate a first substrate and a second substrate spaced a first distance from the first substrate. First thermographic data of the breast tissue is acquired in a first state of compression. The first thermographic data is acquired using a thermal camera. The breast tissue is transitioned from the first state of compression to a second state of compression by changing the first distance. Second thermographic data of the tissue is acquired in the second state of compression. A mammogram of the breast tissue is acquired. The mammogram is acquired while the breast tissue is in at least one of the first state of compression and the second state of compression. The method comprises identifying a cancerous lesion in the breast tissue based on an analysis of the first thermographic data, the second thermographic data, and the mammogram.

Although the present disclosure relates to different aspects and embodiments, it is understood that the different aspects and embodiments disclosed herein can be integrated, combined, or used together as a combination system, or in part, as separate components, devices, and systems, as appropriate. Thus, each embodiment disclosed herein can be incorporated in each of the aspects to varying degrees as appropriate for a given implementation.

These and other features of the applicant's teachings are set forth herein.

### BRIEF DESCRIPTION OF THE FIGURES

Unless specified otherwise, the accompanying drawings illustrate aspects of the innovations described herein. Referring to the drawings, wherein like numerals refer to like parts throughout the several views and this specification, several embodiments of presently disclosed principles are illustrated by way of example, and not by way of limitation. The drawings are not intended to be to scale. A more complete understanding of the disclosure may be realized by reference to the accompanying drawings in which:
FIG. 1A is a schematic diagram of a non-limiting embodiment of a system in accordance with the present disclosure showing the tissue in a first state of compression;
FIG. 1B is a schematic diagram of the system of FIG. 1A showing the tissue in a second state of compression;
FIG. 1C is a schematic diagram of a non-limiting embodiment of a thermal imaging system in accordance with the present disclosure;
FIG. 2 is a schematic diagram of a non-limiting embodiment of a mammography system in accordance with the present disclosure; and
FIG. 3 is a non-limiting embodiment of a method for detecting an area of interest in a tissue according to the present disclosure.

### DETAILED DESCRIPTION

Human tissue undergoes vascular changes during its compression and decompression. For example, as a human breast is compressed, the breast vasculature is compressed, thereby altering the dynamics of blood flow in the breast tissue. Breast lesions have increased vasculature, and the compression process will alter the blood flow in the lesion as well as in the normal breast tissue surrounding the lesion. Decompression of the breast subsequent to compression may be accompanied by a sudden increase in the blood flow which peaks prior to returning to a normal flow. This phenomenon may not be uniform in the presence of breast lesions which create variations in the blood flow. Accordingly, capturing the spatial variations of blood flow dynamics in breast tissue while the tissue is compressed and then decompressed may provide diagnostic information useful for detecting the presence of a lesion. The present inventors believe capturing blood flow dynamics may provide diagnostic insight into tissue in other body regions, such as in limbs where blood inflow following a decompression thereof may be indicative of a wound healing status.

Current mammography examinations involve a compression of breast tissue between a compression paddle and an X-ray detector housing followed by one of more X-ray exposures. During a compression phase, the physical shape of the breast is altered via compression until the breast is flattened to a generally uniform thickness facilitating an X-ray exposure with a fixed X-ray technique. Following the X-ray exposure, the compressive force on breast tissue is released in a decompression phase. In general, X-ray exposures are most effective when the breast is compressed to a generally uniform thickness. Mammography exams generally do not provide any diagnostic information derived during the transition between the compression and decompression phases.

The present inventors determined that utilizing thermal imaging techniques may provide an enhanced diagnostic insight, particularly for patients with high breast density where the cancer detection rate of X-ray imaging is lower. Thus, the present disclosure includes a novel method for detecting an area of interest in a tissue. The method comprises acquiring first thermographic data of the tissue in a first state of compression. The method comprises increasing or decreasing a compressive force applied to the tissue to transition the tissue from the first state of compression to a second state of compression different than the first state of compression, and acquiring second thermographic data of the tissue in the second state of compression. The method comprises determining an area of interest based on an analysis of the first thermographic data and the second thermographic data.

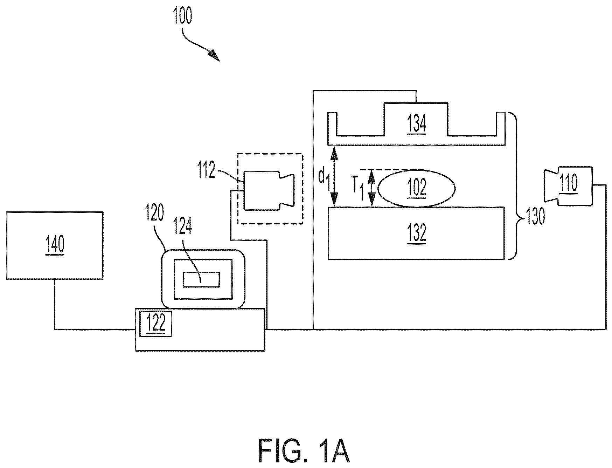

FIG. 1A illustrates a non-limiting embodiment of a system 100 for detecting an area of interest in a tissue in accordance with the present disclosure. The system 100 comprises a thermal camera 110 and a control circuit 120 in signal communication with the thermal camera 110. The thermal camera 110 can be capable to acquire thermographic data of an object 102, and the control circuit 120 can be capable to determine an area of interest of the object 102 based on an analysis of the thermographic data.

The thermal imaging system 100 may optionally comprise a tissue compression system 130 for compressing the object 102. The tissue compression system 130 can comprise a first substrate 132 and a second substrate 134 spaced from the first substrate 132 by a distance d₁.

The object 102 may be a compressible portion of tissue of a subject, such as, for example, a human or an animal. For example, the object 102 may be human breast tissue. The object 102 may be compressed at a point of care (e.g., a clinic or a hospital).

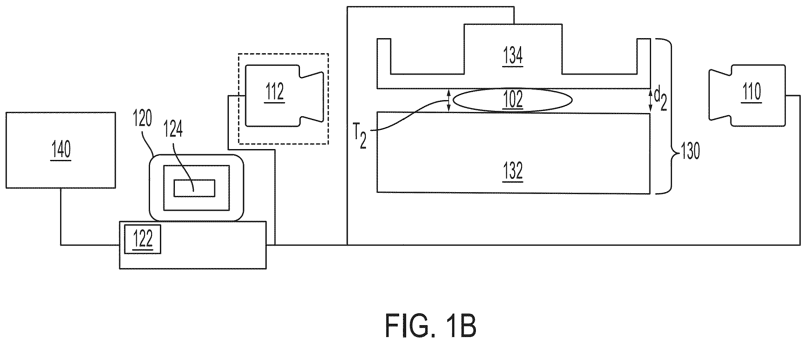

The object 102 may be positioned intermediate a first substrate 132 and a second substrate 134 separated by distance, d₁, as illustrated in FIG. 1A. The first substrate 132 and the second substrate 134 can be moveable relative to one another between the distance, d₁, in FIG. 1A to the distance, d₂, in FIG. 1B. For example, during a compression phase of the compression cycle, the first substrate 132 and/or the second substrate 134 may be moved relative to one another to decrease distance, d₁, as illustrated in FIG. 1A to distance, d₂, as illustrated in FIG. 1B, to thereby increase a compressive force to the object 102 disposed between the substrates 132, 134. During a decompression phase of the compression cycle, the first substrate 132 and/or the second substrate 134 may be moved relative to one another to increase distance, d₂, as illustrated in FIG. 1B, to distance, d₁, as illustrated in FIG. 1A, thereby reducing a compressive force applied to the object 102 disposed between the substrates 132, 134. When minimal, if any, compressive force is being applied to the object 102, a region of object 102 in contact with one or both of the first substrate 132 and second substrate 134 may be minimal such that the object 102 undergoes substantially no deformation, thereby defining an initial state of a compression cycle.

During the compression cycle, a thickness, T₁, of the object 102 as illustrated in FIG. 1A can change to a thickness, T₂, of the object 102 as illustrated in FIG. 1B. For example, during the compression phase, the thickness, T₁, can be reduced to thickness, T₂. While compressed, the object 102 may be a substantially uniform thickness, and the thickness, T₂, can be substantially the same as the distance, d₂, between substrates 132, 134, as illustrated in FIG. 1B. During the compression cycle, one or more compression phases and one or more decompression phases can occur to obtain various states of compression.

The thermal camera 110 can capture thermographic data discretely (e.g., capturing a single thermal image), continuously (e.g., a series or burst of thermal images captured over a pre-determined period of time, and/or a thermal video), or both discretely and continuously. Parameters of the thermal camera 110, such as, for example, focal length, frame rate, and spectral response, can be configured to optimize thermal image capture for a range of temperatures associated with the object 102. For example, when the object 102 is human breast tissue, the thermal camera 110 can comprise a focal length configured to capture thermal images of tissue positioned within 0.5 meters of the thermal camera. In various non-limiting embodiments, the focal length can be less than 100 millimeters, such as, for example, less than 80 millimeters, less than 60 millimeters, less than 50 millimeters, less than 40 millimeters, less than 30 millimeters, or less than 20 millimeters.

The thermal camera 110 can be configured to capture dynamic changes in blood flow, such as, for example, in breast tissue. In various non-limiting embodiments, the thermal camera 110 can have a frame rate of at least 5 frames per second, such as, for example, at least 10 frames per second, at least 15 frames per second, or at least 20 frames per second. In one non-limiting embodiment, the thermal camera 110 may be configured to acquire thermal images of the object 102 at a rate of at least 15 frames per second, which may be useful for uncovering anomalous behavior in blood flow dynamics of high vasculature and/or high density tissues, such as breast tissue.

The spectral response of the thermal camera 110 can be in a range for detecting emissions of thermal radiation. In various non-limiting embodiments, the thermal camera 110 can be a middle-wave infrared (MWIR) camera or a long-wave infrared (LWIR) camera. In various non-limiting embodiments, the thermal camera 110 can have a spectral response in a range of 1 microns to 15 microns, such as, for example, 3 microns to 5 microns, or 7 microns to 14 microns.

The thermal camera 110 can be configured to capture small variations in tissue temperature which may be specific to changes in blood flow. In various non-limiting embodiments, the thermal camera 110 can have a thermal sensitivity of no greater than 60 milliKelvins (mK), such as, for example, no greater than 50 mK, no greater than 40 mK, no greater than 35 mK, no greater than 30 mK, no greater than 25 mK, or no greater than 18 mK. As used herein, the term "thermal sensitivity" is used in reference to a Noise Equivalent Temperature Difference evaluated at 30°C.

Referring yet again to FIG. 1A, the angular extent of an area observed by the thermal camera 110 can be defined by a field of view ("FOV"). The object 102 can be at least partially positioned within the FOV during the compression cycle. The position of the thermal camera 110 can be fixed or dynamically adjustable, provided that the FOV of the thermal camera 110 can capture thermographic data for a portion of the object 102 suitable for detecting an area of interest. The FOV can be configured to capture thermographic data of tissue within a distance of 0.5 m from the thermal camera 110. In various non-limiting embodiments, the thermal camera 110 can have a FOV greater than 15 degrees, such as, for example, greater than 20 degrees, greater than 25 degrees, greater than 30 degrees, greater than 35 degrees, greater than 40 degrees, or greater than 45 degrees.

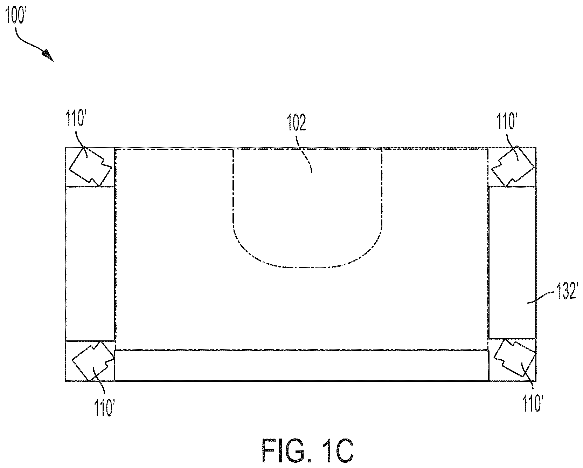

In various non-limiting embodiments, the system 100 may comprise more than one thermal camera 110 to capture, from multiple vantage points, thermographic data for a given state of compression of the object 102. For example, the system 100 may include at least two thermal cameras 110, 112 opposing one another, as illustrated in FIGs. 1A and 1B. In various non-limiting embodiments, the system 100' can comprise four thermal cameras 110' as illustrated in FIG. 1C.

Utilizing multiple thermal cameras 110' can acquire thermographic data of various portions of the object 102 and/or overlapping portions from various vantages points. When the object 102 is breast tissue, the multiple the views of the surface of the breast tissue may enhance the thermal imaging data. For example, a FOV of one of the thermal cameras 110' may not capture all portions of the breast tissue that are desirable to be interrogated. In various examples, multiple vantage points may enhance the detection of hot spots and/or potential lesions when using a neural network for analysis of the images.

In certain non-limiting embodiments, a thermal camera may be incorporated into a substrate of the system 100. Referring to FIG. 1C, the system 100' can comprise thermal cameras 110' incorporated into a first substrate 132' such that each of the thermal cameras 110' are positioned beneath a surface of the substrate 132' for contacting an object 102. Each thermal camera 110' can be angled away from the substrate towards the object 102 such that at least a portion of the object 102 is in a FOV of each thermal camera 110'.

Referring again to FIGs. 1A and 1B, the control circuit 120 can be in signal communication with the thermal camera 110, the thermal camera 112, if present, and the tissue compression system 130 such that the capture of thermographic data is coordinated with tissue compression by the control circuit 120. For example, the control circuit 120 may receive, from an output circuit of the tissue compression system 130, data indicative of a first state of compression of object 102 and may send a control signal to an input circuit of the tissue compression system 130 to transition the object 102 from the first state of compression to a second state of compression.

The control circuit 120 may send a control signal to an input circuit of thermal camera 110 to capture thermographic data which may be based on the status of the tissue compression system 130 and/or the state of compression of the object 102 as described herein. In various non-limiting embodiments, the control circuit 120 may also include software and/or hardware interfaces for facilitating signal communication with other embedded systems, such as, for example, a control circuit of an X-ray mammography system. For example, the control circuit 120 can include a software development kit (SDK), an application programming interface (API), and/or other software-based utilities for interfacing with external systems.

As used herein, the term "control circuit" may refer to, for example, hardwired circuitry, programmable circuitry (e.g., a computer processor comprising one or more individual instruction processing cores, processing unit, processor, microcontroller, microcontroller unit, controller, digital signal processor (DSP), programmable logic device (PLD), programmable logic array (PLA), or FPGA), state machine circuitry, firmware that stores instructions executed by programmable circuitry, and any combination thereof. The control circuit 120 may, be embodied, collectively or individually, as circuitry that forms part of a larger system, for example, an IC, an ASIC, a SoC, a desktop computer, a laptop computer, a tablet computer, a server, a smart phone, etc. Accordingly, as used herein, a "control circuit" can comprise electrical circuitry having at least one discrete electrical circuit, electrical circuitry having at least one IC, electrical circuitry having at least one application-specific IC, electrical circuitry forming a general-purpose computing device configured by a computer program (e.g., a general-purpose computer configured by a computer program that at least partially carries out processes and/or devices described herein or a microprocessor configured by a computer program that at least partially carries out processes and/or devices described herein), electrical circuitry forming a memory device (e.g., forms of RAM), and/or electrical circuitry forming a communications device (e.g., a modem, communications switch, or optical-electrical equipment). The subject matter described herein may be implemented in an analog or digital fashion, or some combination thereof.

Referring again to FIGs. 1A and 1B, the control circuit 120 may synchronize tissue compression and thermographic data acquisition. For example, the control circuit 120 may control data capture by the thermal camera 110 based on a state of compression of the object 102 such that the thermal camera 110 captures thermographic data of the object 102 at a first state of compression and at a second state of compression. The first and second states of compression can correspond to different compressive forces and/or different tissue thicknesses.

The thermal camera 110 may intermittently and/or continuously capture thermographic data of the object 102 throughout a compression cycle. The thermographic data may be linked and/or stored with corresponding tissue compression data such as tissue thickness, and operational status of the compression system, e.g., compression phase or decompression phase. The thermographic data may be stored locally, such as, for example, within memory 122 of the control circuit 120, and/or stored remotely, such as, for example, via uploading to a cloud-based storage. The thermographic data may also be transferred, via the control circuit 120, to another system, such as, for example, a mammography system if the system 100 is incorporated into an OEM x-ray imaging system, or a hospital PACS (Picture Archiving and Communications System) if the thermographic data is coded in a DICOM (Digital Imaging and Communications in Medicine) format.

The thermal camera 110 may capture thermographic data at a discrete time or timestamp, which may be used to produce a static thermal image. The thermal camera 110 may capture thermographic data over time to form a sequential set of data. A sequential set of data can comprise a sequence of static thermal images and/or a thermal video captured over a continuous range of time.

The captured thermographic data can be associated with a static event, wherein a particular state of compression is held for a period of time, or a dynamic event, wherein tissue is actively undergoing a compression cycle (i.e., is subjected to a change in compressive force). In various embodiments, the control circuit 120 may cause the thermal camera 110 to capture thermographic data of the object 102 for a period of time that the tissue is at a particular tissue thickness, a particular distance between substrates 132, 134, and/or dynamically across thicknesses and/or distances. For example, the control circuit 120 may trigger the thermal camera 110 to capture a set of thermographic data of the object 102 from a first time when the object 102 is compressed to thickness, T₂, as illustrated in FIG. 1B, and hold the tissue compression system 130 in this state to maintain thickness, T₂, until a second time at which the thermal camera 110 may stop capturing thermographic data in the current set and/or start capturing thermographic data in a subsequent set beginning with the second time. Acquiring thermographic data in this manner may capture changes in blood flow within the object 102 during a static compression.

In various non-limiting embodiments, thermographic data may be captured over all or a portion of a compression cycle of the object 102. For example, the control circuit 120 may trigger capture of thermographic data during a compression phase from a first time when the object 102 is in a first state of compression as illustrated in FIG. 1A, and throughout a compression phase of the object 102 to a second state of compression corresponding to thickness, T₂, as illustrated in FIG. 1B. In certain non-limiting embodiments, the control circuit 120 may trigger capture of thermographic data during a decompression phase where the object 102 at thickness, T₂, as illustrated in FIG. 1B is released until reaching initial thickness, T₁, as illustrated in FIG. 1A. In certain non-limiting embodiments, the control circuit 120 may trigger capture of thermographic data during the compression phase and the decompression phase.

The control circuit 120 can output thermographic data, such as, for example, to be displayed on a display device 124. The control circuit 120 can be configured to transform the thermographic data to generate a graphical visualization of the object 102 and, optionally, output the graphical visualization to be displayed on the display device 124. For example, the control circuit 120 may generate difference data based on comparison of thermographic data acquired during compression of object 102 and thermographic data acquired during decompression of the object 102. In various embodiments, the graphical visualization may be static, such as an artificial thermal image generated from differences between thermographic data discretely acquired at two separate times but at a common tissue thickness, such as a tissue thickness between thickness, T₁, and thickness, T₂, encountered during both a compression phase and a decompression phase of a common compression cycle.

In various embodiments, the graphical visualization can be dynamic, such as, for example, an artificial thermal video generated from differences between a set of thermographic data acquired during a compression phase of a compression cycle and a set of thermographic data acquired during a decompression phase of the same compression cycle, the differences being evaluated based on tissue thickness.

Referring yet again to FIGs. 1A and 1B, the control circuit 120 can be capable to determine an area of interest of the object 102 based on an analysis of thermographic data. For example, the control circuit 120 may determine an area of interest based on differences in data (e.g., determined based on a comparison of thermographic data of the region at various states of compression). In various non-limiting embodiments, the area of interest may be based on differences in temperature in a portion of the thermographic data. For example, the differences in temperature in an area of interest may be a region of tissue exhibiting anomalous blood flow, which can affect the temperature of the tissue.

In certain non-limiting embodiments, the system 100 may comprise a neural network 140 accessible by (e.g., stored in memory in signal communication with) the control circuit 120. The neural network 140 may be fed thermographic data or a graphical visualization generated from the thermographic data for analysis. The neural network 140 may facilitate a detection of specific features of interest within object 102 based on correlations determined by the neural network 140 during a training phase.

For example, during a training phase, the neural network 140 may be trained with graphical visualizations generated from thermographic data of objects undergoing at least a portion of a compression cycle (e.g., tissue undergoing compression and/or decompression to identify blood flow dynamics as normal or abnormal). For example, when the object 102 is breast tissue 102, the neural network 140 may be trained with clinical data associated with cancerous lesions, such as, for example, outputted by x-ray imaging systems, to validate an area of interest as being cancerous based on spatial characteristics of blood flow within the area. The neural network 140 may be trained with clinical data produced by other diagnostic methods, such as X-ray data from a mammography. Based on an output from the neural network 140, the control circuit 120 may then identify regions of tissue as being of a specific nature, such as cancerous lesions in breast tissue.

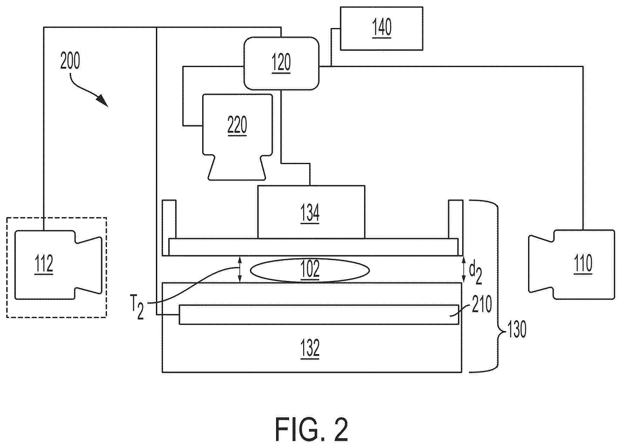

The system 100 may be incorporated into a mammography system to provide multiple sources of diagnostic information from a single compression cycle. For example, FIG. 2 illustrates a non-limiting embodiment of a mammography system 200 in accordance with the present disclosure. The mammography system 200 comprises the system 100 including the thermal camera 110, the control circuit 120, the tissue compression system 130, the neural network 140, and an X-ray imaging system comprising an X-ray detector 210, which may be embedded within the first substrate 132, and an X-ray source 220. The system 200 may provide a comprehensive package for identifying cancerous lesions without requiring the patient to participate in multiple procedures.

The X-ray source 220 can emit X-ray electromagnetic radiation that passes through the object 102 and is received by the X-ray detector 210. The X-ray detector 210 can output X-ray data that can be used to produce an X-ray image.

The control circuit 120 may be in signal communication with the thermal camera 110, tissue compression system 130, X-ray detector, X-ray source 220, and neural network 140 to coordinate compression of the object 102, acquiring thermographic data, and acquiring an X-ray image. For example, the control circuit 120 can receive the X-ray data and produce an X-ray image therefrom. In various non-limiting embodiments, the control circuit 120 can combine the X-ray data with the thermography data to create a hybrid image. The hybrid image can be based on a single compression cycle or multiple compression cycles, depending on the application.

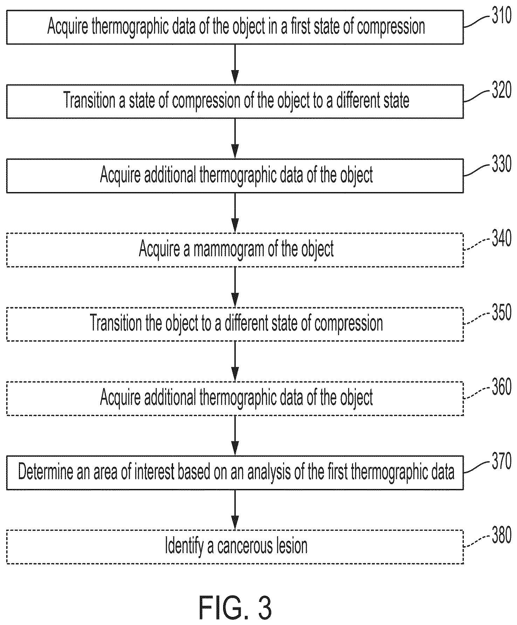

FIG. 3 illustrates a non-limiting embodiment of a method for detecting an area of interest in a tissue in accordance with the present disclosure. The method may be executed with the system 100 described hereinabove. At step 310, the method can comprise acquiring first thermographic data of object 102 in a first state of compression (e.g., the object 102 at a thickness, T₁, as illustrated in FIG. 1A, prior to being compressed in the tissue compression system 130, or the object 102 at the thickness, T₂, as illustrated in FIG. 2A). The first thermographic data can be acquired with the thermal camera 110.

At step 320, the method can comprise transitioning 320 a state compression of the object 102 to a different state. For example, the object 102 can be transitioned from the first state of compression to a second state of compression different than the first state of compression. Additional thermographic data (e.g., second thermographic data) of the object 102 in the second state of compression can be acquired at step 330. The thermographic data, including the first thermographic data and/or the second thermographic data, can be acquired with the thermal camera 110, the thermal camera 112, or at least two thermal cameras (e.g., both thermal cameras 110 and 112).

In various embodiments, transitioning 320 the object 102 from the first state of compression to the second state of compression can comprise changing a compressive force applied to the object 102 positioned intermediate the first substrate 132 and the second substrate 134 in the first state of compression until the object 102 is in a second state of compression. For example, a compressive force applied to the object 102 can be increased by transitioning the distance, d₁, as illustrated in FIG. 1A to the distance, d₂, as illustrated in FIG. 1B. In various non-limiting embodiments, a compressive force applied to the object 102 can be decreased by transitioning the distance, d₂, as illustrated in FIG. 1B to the distance, d₁, as illustrated in FIG. 1A.

Steps 320 and 330 may be repeated as necessary to obtain a desired quantity of thermographic data, a desired state of compression of the object 102, a desired quantity of thermographic data at different states of compression of the object 102.

In various non-limiting embodiments, the method may be executed to determine areas of interest based on observations of object 102 during only a decompression phase, such as, for example, in a wound healing diagnostic application.

The method may also be combined with, or incorporated into, other examination methods. For example, at step 340 the method can optionally comprise acquiring a mammogram (e.g., an X-ray image) of the object 102. Combining thermography with mammography in a single procedure can provide the benefit of providing multiple sources of diagnostic information.

The method can optionally comprise, at step 350, transitioning the tissue to another state of compression, such as, for example, from the second state of compression to a third state of compression different than the first state of compression and the second state of compression. The third state of compression may be the same as the first state of compression, or the third state of compression may be different from the first state of compression.

Additional thermographic data of the object 102 in the third state of compression can be acquired at step 360. Steps 350 and 360 may be repeated as necessary to obtain a desired quantity of thermographic data, a desired state of compression of the object 102, a desired quantity of thermographic data at different states of compression of the object 102.

At step 370, an area of interest can be determined based on an analysis of the thermographic data (e.g., first thermographic data and the second thermographic data). Determining the area of interest within the object 102 can be based on an analysis of the differences between a first subset and a second subset of thermographic data, which may be performed by the control circuit 120.

In various non-limiting embodiments, a graphical visualization produced from the first thermographic data and the second thermographic data can be displayed on the display device 124. The area of interest can be indicated (e.g., mapped) in the graphical visualization by various methods, such as, for example, a bounding box, color, a symbol, or other indicator.

In embodiments where the object is breast tissue, the method 300 may optionally include, at step 380, identifying a cancerous lesion in the breast tissue. The cancerous lesion can be identified as an area of interest based on an analysis of the first thermographic data, the second thermographic data, and/or the mammogram. In various examples, the identification may be performed by a physician reviewing the graphical visualization.

In various examples, identifying a region of interest in the object 102 and/or identifying a cancerous lesion can be performed using a neural network 140. For example, the neural network 140 may identify suspicious regions in the thermography data and/or mammogram based on processing the thermography data, X-ray data, difference data generated therefrom, and/or graphical visualization generated therefrom. In various non-limiting embodiments, the identified cancerous lesion may be indicated in the graphical visualization.

The method can provide the benefit of using multiple sources of diagnostic information to enhance the detection of anomalies (e.g., hot spots, cancerous lesions) in a non-invasive manner.

Although described herein with reference to breast tissue and cancerous lesions, the present disclosure further considers using the thermal imaging system according to the present disclosure for imaging other tissues, such as tissues undergoing a healing process. The resulting image of the healing tissue may be able to evaluate a healing status of the tissue to facilitate a medical diagnosis or prescribe additional testing. Further, the present disclosure may be used to produce a thermal image of any other object, for medical or nonmedical purposes.

The following numbered clauses are directed to various non-limiting embodiments according to the present disclosure:
Clause 1. A method for detecting an area of interest in an object, comprising: acquiring first thermographic data of the object in a first state of compression; transitioning the object from the first state of compression to a second state of compression different than the first state of compression by increasing or decreasing a compressive force applied to the object; acquiring second thermographic data of the object in the second state of compression; determining an area of interest based on an analysis of the first thermographic data and the second thermographic data.
Clause 2. The method of clause 1, wherein transitioning the object from the first state of compression to the second state of compression comprises increasing or decreasing a compressive force applied to the object using a first substrate and a second substrate spaced a first distance apart from the first substrate, wherein the object is positioned intermediate the first substrate and the second substrate in the first state of compression and in the second state of compression.
Clause 3. The method of clause 2, wherein the method comprises transitioning the object from the first state of compression to the second state of compression by decreasing the first distance and thereby increasing a compressive force applied to the object.
Clause 4. The method of clause 2, wherein the method comprises transitioning the object from the first state of compression to the second state of compression by increasing the first distance and thereby reducing a compressive force applied to the object.
Clause 5. The method of any of clauses 1-4, wherein acquiring second thermographic data of the object in the second state of compression comprises: maintaining the object in the second state of compression for a period of time after transitioning the object to the second state of compression; and acquiring the second thermographic data after the period of time.
Clause 6. The method of any of clauses 1-5, further comprising transitioning the object from the second state of compression to a third state of compression different than the first state of compression and the second state of compression by increasing or decreasing a compressive force applied to the object.
Clause 7. The method of clause 6, further comprising acquiring a mammogram of the object in the second state of compression and wherein determining the area of interest is based on an analysis of the first thermographic data, the second thermographic data, and the mammogram.
Clause 8. The method of any of clauses 1-7, further comprising displaying a graphical visualization produced from the first thermographic data and the second thermographic data.
Clause 9. The method of clause 8, further comprising acquiring a set of thermographic data comprising the first thermographic data and the second thermographic data, wherein the graphical visualization comprises a difference between subsets of the set of thermographic data.
Clause 10. The method of clause 9, wherein the difference is a static difference.
Clause 11. The method of clause 9, wherein the difference is a dynamic difference.
Clause 12. The method of any of clauses 9-11, wherein the subsets of the set of thermographic data comprise: a first subset of thermographic data for a compression phase comprising the first state of compression; and a second subset of thermographic data for a decompression phase comprising the second state of compression.
Clause 13. The method of clause 12, wherein determining the area of interest within the object is based on an analysis of the differences between the first subset and the second subset of the thermographic data.
Clause 14. The method of any of clauses 9-13, wherein acquiring the set of thermographic data comprises acquiring thermal images of the object at a rate of at least 15 frames per second.
Clause 15. The method of any of clauses 1-14, wherein the object is breast tissue and further comprising identifying a cancerous lesion based on the analysis of the first thermographic data and the second thermographic data using a neural network trained to detect characteristics of cancerous lesions.
Clause 16. The method of clause 15, wherein indicating the identified cancerous lesion comprises displaying a graphical visualization produced from the first thermographic data and the second thermographic data visualization.
Clause 17. A thermal imaging system capable to perform the method of any of clauses 1-16, the thermal imaging system comprising: a thermal camera capable to acquire the first thermographic data of the object and acquire the second thermographic data of the object; and a control circuit in signal communication with the thermal camera, the control circuit capable to determine the area of interest based on the analysis of the first thermographic data and the second thermographic data.
Clause 18. The thermal imaging system of clause 17, wherein the thermal camera comprises: a focal length configured to capture thermal images of an object positioned within 0.5 meters of the thermal camera; a frame rate of at least 15 frames per second; and a spectral response in a range of 3 microns to 15 microns.
Clause 19. The thermal imaging system of any of clauses 17-18, wherein the object is breast tissue and further comprising a neural network trained to detect characteristics of cancerous lesions, wherein the control circuit is configured to access the neural network to identify the cancerous lesions.
Clause 20. A mammography system comprising the thermal imaging system of any of clauses 17-19.
Clause 21. A method for detecting a cancerous lesion in breast tissue, comprising: disposing the breast tissue intermediate a first substrate and a second substrate spaced a first distance from the first substrate; acquiring first thermographic data of the breast tissue in a first state of compression using a thermal camera; transitioning the breast tissue from the first state of compression to a second state of compression by changing the first distance; acquiring second thermographic data of the tissue in the second state of compression; acquiring a mammogram of the breast tissue while in at least one of the first state of compression and the second state of compression; and identifying a cancerous lesion based on an analysis of the first thermographic data, the second thermographic data, and the mammogram.

Having thus described several aspects and embodiments of the technology of this application, it is to be appreciated that various alterations, modifications, and improvements will readily occur to those of ordinary skill in the art. Such alterations, modifications, and improvements are intended to be within the scope of the technology described in the application. It is, therefore, to be understood that the foregoing embodiments are presented by way of example only and that, within the scope of the appended claims and equivalents thereto, inventive embodiments may be practiced otherwise than as specifically described. In addition, any combinations of two or more features, systems, articles, materials, and/or methods described herein, if such features, systems, articles, materials, and/or methods are not mutually inconsistent, are included within the scope of the present disclosure.

In certain embodiments, a processor may be a physical or virtual processor. In other embodiments, a virtual processor may be spread across one or more portions of one or more physical processors. In certain embodiments, one or more of the embodiments described herein may be embodied in hardware such as a Digital Signal Processor (DSP). In certain embodiments, one or more of the embodiments herein may be executed on a DSP. One or more of the embodiments herein may be programmed into a DSP. In some embodiments, a DSP may have one or more processors and one or more memories. In certain embodiments, a DSP may have one or more computer readable storages. In many embodiments, a DSP may be a custom designed ASIC chip. In other embodiments, one or more of the embodiments stored on a computer readable medium may be loaded into a processor and executed.

Also, as described, some aspects may be embodied as one or more methods. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

The phrase "and/or", as used herein in the specification and in the claims, should be understood to mean "either or both" of the elements so conjoined, i.e., elements that are conjunctively present in some cases and disjunctively present in other cases.

As used herein in the specification and in the claims, the phrase "at least one", in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "carrying," "having," "containing," "involving," "holding," "composed of," and the like are to be understood to be open-ended, i.e., to mean including but not limited to. The transitional phrases "consisting of" and "consisting essentially of" shall be closed or semi-closed transitional phrases, respectively.

Where a range or list of values is provided, each intervening value between the upper and lower limits of that range or list of values is individually contemplated and is encompassed within the disclosure as if each value were specifically enumerated herein. In addition, smaller ranges between and including the upper and lower limits of a given range are contemplated and encompassed within the disclosure. The listing of exemplary values or ranges is not a disclaimer of other values or ranges between and including the upper and lower limits of a given range.

Embodiments disclosed herein may be embodied as a system, method, or computer program product. Accordingly, embodiments may take the form of an entirely hardware embodiment, an entirely software embodiment (including firmware, resident software, microcode, etc.), or an embodiment combining software and hardware aspects that may all generally be referred to herein as a "circuit", "module", or "system". Furthermore, embodiments may take the form of a computer program product embodied in one or more computer readable medium(s) having computer readable program code embodied thereon.

## Claims

1. A method for detecting an area of interest in an object, comprising:
acquiring first thermographic data of the object in a first state of compression;
transitioning the object from the first state of compression to a second state of compression different than the first state of compression by increasing or decreasing a compressive force applied to the object;
acquiring second thermographic data of the object in the second state of compression;
determining an area of interest based on an analysis of the first thermographic data and the second thermographic data.

2. The method of claim 1, wherein transitioning the object from the first state of compression to the second state of compression comprises increasing or decreasing a compressive force applied to the object using a first substrate and a second substrate spaced a first distance apart from the first substrate, wherein the object is positioned intermediate the first substrate and the second substrate in the first state of compression and in the second state of compression.

3. The method of claim 2, wherein the method comprises transitioning the object from the first state of compression to the second state of compression by decreasing the first distance and thereby increasing a compressive force applied to the object, or
wherein the method comprises transitioning the object from the first state of compression to the second state of compression by increasing the first distance and thereby reducing a compressive force applied to the object.

4. The method of any of claims 1-3, wherein acquiring second thermographic data of the object in the second state of compression comprises:
maintaining the object in the second state of compression for a period of time after transitioning the object to the second state of compression; and
acquiring the second thermographic data after the period of time.

5. The method of any of claims 1-4, further comprising
transitioning the object from the second state of compression to a third state of compression different than the first state of compression and the second state of compression by increasing or decreasing a compressive force applied to the object,
wherein optionally the method further comprises acquiring a mammogram of the object in the second state of compression and wherein determining the area of interest is based on an analysis of the first thermographic data, the second thermographic data, and the mammogram.

6. The method of any of claims 1-5, further comprising displaying a graphical visualization produced from the first thermographic data and the second thermographic data.

7. The method of claim 6, further comprising acquiring a set of thermographic data comprising the first thermographic data and the second thermographic data, wherein the graphical visualization comprises a difference between subsets of the set of thermographic data.

8. The method of claim 7, wherein the difference is a static difference, or wherein the difference is a dynamic difference.

9. The method of claim 7 or 8, wherein the subsets of the set of thermographic data comprise:
a first subset of thermographic data for a compression phase comprising the first state of compression; and
a second subset of thermographic data for a decompression phase comprising the second state of compression.
wherein optionally determining the area of interest within the object is based on an analysis of the differences between the first subset and the second subset of the thermographic data.

10. The method of any of claims 7-9, wherein acquiring the set of thermographic data comprises acquiring thermal images of the object at a rate of at least 15 frames per second.

11. The method of any of claims 1-10, wherein the object is breast tissue and further comprising identifying a cancerous lesion based on the analysis of the first thermographic data and the second thermographic data using a neural network trained to detect characteristics of cancerous lesions,
wherein optionally indicating the identified cancerous lesion comprises displaying a graphical visualization produced from the first thermographic data and the second thermographic data visualization.

12. A thermal imaging system capable to perform the method of any of claims 1-11, the thermal imaging system comprising:
a thermal camera capable to acquire the first thermographic data of the object and acquire the second thermographic data of the object; and
a control circuit in signal communication with the thermal camera, the control circuit capable to determine the area of interest based on the analysis of the first thermographic data and the second thermographic data.

13. The thermal imaging system of claim 12, wherein the thermal camera comprises:
a focal length configured to capture thermal images of an object positioned within 0.5 meters of the thermal camera;
a frame rate of at least 15 frames per second; and
a spectral response in a range of 3 microns to 15 microns,
and/or wherein the object is breast tissue and the thermal imaging system further comprises a neural network trained to detect characteristics of cancerous lesions, wherein the control circuit is configured to access the neural network to identify the cancerous lesions.

14. A mammography system comprising the thermal imaging system of claim 12 or 13.

15. A method for detecting a cancerous lesion in breast tissue, comprising:
disposing the breast tissue intermediate a first substrate and a second substrate spaced a first distance from the first substrate;
acquiring first thermographic data of the breast tissue in a first state of compression using a thermal camera;
transitioning the breast tissue from the first state of compression to a second state of compression by changing the first distance;
acquiring second thermographic data of the tissue in the second state of compression;
acquiring a mammogram of the breast tissue while in at least one of the first state of compression and the second state of compression; and
identifying a cancerous lesion based on an analysis of the first thermographic data, the second thermographic data, and the mammogram.
